# EUROPÄISCHE PATENTANMELDUNG

(11) **EP 1 417 962 A1**
(43) Veröffentlichungstag der Anmeldung: **12.05.2004**
(21) Anmeldenummer: 03450245.0
(22) Anmeldetag: 05.11.2003
(51) Int. Cl.: A61K 31/215

(54) **Verfahren zur Herstellung eines Arzneimittels enthaltend Esmolol**

(30) Priorität: 06.11.2002 AT 16672002
(71) Anmelder: AOP Orphan Pharmaceuticals AG, 1160 Wien (AT)
(72) Erfinder: Widmann, Rudolf, Stefan, 3002 Purkersdorf (AT)
(74) Vertreter: Nemec, Harald, Dipl.-Ing.

(57) **Zusammenfassung**

Die Erfindung betrifft. Verfahren zur Herstellung eines Arzneimittels in flüssiger Form enthaltend Esmolol oder ein pharmazeutisch verträgliches Salz, Solvat oder Derivat davon. Das erfindungsgemäße Verfahren umfaßt die Schritte
a) Herstellen einer Verdünnung von Esmolol oder eines pharmazeutisch verträglichen Salzes, Solvates oder Derivates davon in einem pharmazeutisch verträglichen Verdünnungsmittel,
b) Abfüllen der Verdünnung in ein geeignetes Gefäß,
c) wobei die Schritte a) und b) so durchgeführt werden, daß das Gefäß 30 ml bis 70 ml Verdünnung mit einem Anteil von 1500 mg bis 3500 mg Esmolol oder des pharmazeutisch verträglichen Salzes, Solvates oder Derivates davon enthält,
d) Überführen des Gefäßes in eine verkaufsfertige Form.

## Beschreibung

Die vorliegende Erfindung betrifft ein Verfahren zur Herstellung eines Arzneimittels in flüssiger Form enthaltend Esmolol oder ein pharmazeutisch verträgliches Salz, Solvat oder Derivat davon.

Esmolol ist ein Kurzzeit-beta-adrenerger-Blocker und wird in der Behandlung oder Vorbeugung von Herzstörungen verwendet (US 4,387,103 A).

Esmolol wird in verschiedenen akuten Indikationen eingesetzt:
Behandlung von Sinus-Tachycardia
Behandlung von tachycardischer Atrio-Arrhythmie
Behandlung von ventrikulärer Arryhthmie
Behandlung von Hypertonie
Behandlung von myokardialer Ischämie
Behandlung von perioperativer Ischämie.

In der Beschreibung der EP 0 053 435 B1 wird erwähnt, daß Esmolol in einer Dosierung von 0,001-100 mg/kg Körpergewicht angewendet werden kann.

Die EP 0 403 578 B1 beansprucht in Anspruch 1 eine injizierbare, wäßrige Zusammensetzung zur Behandlung von Herzzuständen, die eine effektive Menge Esmolol (Hydrochlorid) in einem Anteil von 1 mg bis 250 mg Esmolol/ml Lösung und 0,01 bis 0,04 M Puffer umfaßt sowie einen pH-Wert im Bereich von 4,5 bis 5,5 aufweist.

In der Offenbarung der EP 0 403 578 B1 wird auf Seite 3 eine Verdünnung mit 250 mg Esmolol/ml Lösung erwähnt. Weiters wird auf Seite 6 (Zeile 31) bzw. in Tabelle 1 eine Verdünnung mit 50 mg Esmolol/ml Lösung erwähnt.

Die Internationale Anmeldung WO 02/076446 beschreibt eine Esmolol-Lösung, die 0,1 bis 500 mg/ml Esmolol-Hydrochlorid, 0,01 bis 2 M Puffer und 1 bis 500 mg/ml eines Osmoseeinstellenden Mittels enthält.

Derzeit sind zwei Esmolol enthaltende Präparationen im Handel erhältlich: Eine "ready-touse"-Ampulle mit 10 ml Verdünnung enthaltend 100 mg Esmolol und eine Ampulle mit 10 ml Verdünnung enthaltend 2500 mg Esmolol, welche zur Verdünnung mit 240 ml isotonischer Salzlösung bestimmt ist.

Die Verdünnung mit Salzlösung wird empfohlen, da Esmolol und die übrigen Bestandteile in der Ampulle mit 10 ml/2500 mg Esmolol-Verdünnung die venösen Gefäßwände entzünden.

Um Irrtümer zu vermeiden, ist es üblich, Verdünnungen mit größeren bzw. geringeren Gehalten an Wirkstoff durch die Dimensionierung der Präparation zu unterscheiden. Dies ist bei den derzeit bekannten Präparationen mit Esmolol nicht der Fall, da sie beide 10 ml Verdünnung enthalten.

Es ist daher schon zu Irrtümern gekommen, aufgrund welchen die 10 ml/2500 mg Esmolol-Verdünnung für die "ready-to-use"- 10 ml/100 mg-Verdünnung gehalten wurde. Dadurch wurden Patienten auf einmal 2500 mg Esmolol in einer einzigen Dosis verabreicht, was zu schweren Schäden bei den Patienten bis hin zum Tod führte.

Aus diesem Grund sind manche Ärzte dazu übergegangen, aus der 10 ml/2500 mg Esmolol-Verdünnung eine Präparation mit 50 ml Verdünnung enthaltend 2500 mg Esmolol herzustellen. Damit handeln diese Ärzte jedoch gegen die Empfehlung im Datenblatt, gemäß welcher die 10 ml/2500 mg Esmolol-Verdünnung auf 240 ml verdünnt werden soll.

Die vorliegende Erfindung stellt sich die Aufgabe, ein Verfahren zur Herstellung einer "ready-to-use"-Verdünnung enthaltend Esmolol zur Verfügung zu stellen, mit welchem die oben geschilderten Nachteile überwunden werden sollen.

Diese Aufgabe wird durch ein Verfahren zur Herstellung eines Arzneimittels in flüssiger Form enthaltend Esmolol oder ein pharmazeutisch verträgliches Salz, Solvat oder Derivat davon gelöst, welches die Schritte
a) Herstellen einer Verdünnung von Esmolol oder eines pharmazeutisch verträglichen Salzes, Solvates oder Derivates davon in einem pharmazeutisch verträglichen Verdünnungsmittel,
b) Abfüllen der Verdünnung in ein geeignetes Gefäß,
c) wobei die Schritte a) und b) so durchgeführt werden, daß das Gefäß 30 ml bis 70 ml Verdünnung mit einem Anteil von 1500 mg bis 3500 mg Esmolol des pharmazeutisch verträglichen Salzes, Solvates oder Derivates davon enthält, und
d) Überführen des Gefäßes in eine verkaufsfertige Form
umfaßt.

Gemäß bevorzugten Ausführungsformen des erfindungsgemäßen Verfahrens enthält das Gefäß
- 30 ml Verdünnung mit einem Anteil von 1500 mg Esmolol oder des pharmazeutisch verträglichen Salzes, Solvates oder Derivates davon,
- 50 ml Verdünnung mit einem Anteil von 2500 mg Esmolol oder des pharmazeutisch verträglichen Salzes, Solvates oder Derivates davon oder
- 70 ml Verdünnung mit einem Anteil von 3500 mg Esmolol oder des pharmazeutisch verträglichen Salzes, Solvates oder Derivates davon.

Besonders bevorzugt ist eine 50 ml Verdünnung mit einem Anteil von 2500 mg Esmolol. Es sind aber auch Verdünnungsmengen (ml) mit einem Gehalt an Esmolol (mg) bzw. des pharmazeutisch verträglichen Salzes, Solvates oder Derivates davon denkbar, die zwischen den bevorzugt angegebenen Kombinationen liegen.

Unter dem Begriff "Überführen des Gefäßes in eine verkaufsfertige Form" sind die in der kommerziellen Herstellung von Arzneimittel üblichen Schritte wie z.B. Verpacken, Aufkleben von Etiketten, etc. zu verstehen.

Die durch das erfindungsgemäße Verfahren hergestellten Verdünnungen, insbesondere die 50 ml Verdünnung mit einem Anteil von 2500 mg Esmolol haben die folgenden Vorteile:

Die Verdünnungen können als "ready-to-use" bezeichnet werden - insbesondere bei Einsatz in einer 50 ml-Spritze zur Administration über eine Perfusorpumpe unter Verwendung von Linien, welche mit größeren Venen verbunden sind.

Bei der derzeit empfohlenen 250 ml-Verdünnung ergeben sich außerdem folgende Nachteile:

Es muß zuviel Volumen in Patienten in kritischem Zustand per Infusion verabreicht werden, wenn Esmolol auf eine 250 ml-Lösung verdünnt wird. Diese Verdünnung kann dazu führen, daß einem in intensiver Betreuung befindlichen Patienten bis zu 2000 ml zusätzliches Volumen während 24 h verabreicht werden.

Es ist notwendig, die Volumensituation in akuten Situation wie z.B. myokardialer Ischämie so genau wie möglich zu überwachen, um eine Volumensbelastung für das Herz und damit das Entstehen von Lungenödemen zu verhindern. Die Volumenkontrolle einer 250 ml-Verdünnung ist sehr kompliziert oder sogar unmöglich.

Wenn hingegen eine 50 ml-Verdünnung verwendet wird, müssen in 24 h lediglich 200 ml zusätzliches Volumen verabreicht werden, eine Größenordnung, die hinsichtlich der Volumenskontrolle praktisch vernachlässigt werden kann.

Esmolol wurde insbesondere zur Verwendung in akuten Situation und Notfällen entwickelt. Viele Operationssäle, Wiederbelebungsräume und Notwägen sind nur mit Perfusorpumpen für 50 ml-Spritzen, nicht aber mit Rollenpumpen, welche ein größeres Volumen (250 ml) transportieren können, ausgestattet.

Die Ärzte sind daher gezwungen, gegen die Empfehlung im Datenblatt eine 50 ml/2500 mg Esmolol-Verdünnung zu verwenden.

Die 10 ml/ 100 mg Esmolol-Präparation stellt dazu keine Alternative dar, da sie nur für 6-10 Minuten Beta-Blockade andauert und somit für eine Kurzzeit-Beta-Blockade in Stress-Situationen, wie z.B. dem Beginn einer Anästhesie, Intubation oder der Beendigung der Anästhesie entwickelt wurde.

Das Zur-Verfügung-Stellen einer "ready-to-use" 50 ml/2500 mg Esmolol-Verdünnung erspart im Vergleich zum notwendigen Verdünnen der handelsüblichen 10 ml/2500 mg Esmolol-Verdünnung mit 40 ml Salzlösung wichtige Zeit.

Es ist weiters hygienischer, eine einzige Wirkstoff-Quelle für eine 50-ml-Spritze zu verwenden, anstelle zwei Quellen, nämlich zunächst eine Ampulle, die geöffnet werden muß, und ein Gefäß mit 250 ml Salzlösung, welches bis auf 10 ml Salzlösung geleert werden muß, um eine 250 ml/2500 mg-Verdünnung zu erhalten. Die Wahrscheinlichkeit von mikrobieller Kontamination in klinischer Praxis wird mit der erfindungsgemäß hergestellten 50 ml/2500 mg-Verdünnung stark verringert.

Die erfindungsgemäß hergestellte 50 ml/2500 mg-Verdünnung kann mit weiteren 200 ml Salzlösung zu einer 250 ml-Präparation in einer Flasche oder einem Sack verdünnt werden, wenn eine Verabreichung über eine größere Rollenpumpe wie z.B. dem Infusomat gewählt wird, weil z.B. nur kleinere Venen zugänglich sind und/oder die Volumenskontrolle nicht wesentlich ist.

Die Verwendung der erfindungsgemäß hergestellten 50 ml/2500 mg-Verdünnung verringert die Wahrscheinlichkeit von Glassbrüchen in Notsituationen. Das derzeit notwendige Öffnen der handelsüblichen 10 ml/2500 mg-Ampulle kann nämlich zu Glasbrüchen und Verletzungen führen.

Die erfindungsgemäß hergestellte 50 ml/2500 mg-Verdünnung verringert zudem die Wahrscheinlichkeit von Fehlern, die - wie oben erwähnt - derzeit aufgrund der Verwechslung zwischen der 10 ml/2500 mg-Ampulle und der 10 ml/100 mg-Ampulle vorkommen.

Selbst wenn bei der Verwendung der erfindungsgemäß hergestellten 50 ml/2500 mg-Verdünnung ein Fehler vorkommen sollte, erhöhen sich die Überlebenschancen: Die direkte Injektion von 10 ml dieser Lösung führt zu einer Esmolol-Belastung von 500 mg, während die direkte Injektion von 10 ml der derzeit üblichen 10 ml/2500 mg-Lösung zu einer Esmolol-Belastung von 2500 mg führt.

## Patentansprüche

1. Verfahren zur Herstellung eines Arzneimittels in flüssiger Form enthaltend Esmolol oder ein pharmazeutisch verträgliches Salz, Solvat oder Derivat davon, umfassend die Schritte
a) Herstellen einer Verdünnung von Esmolol oder eines pharmazeutisch verträglichen Salzes, Solvates oder Derivates davon in einem pharmazeutisch verträglichen Verdünnungsmittel,
b) Abfüllen der Verdünnung in ein geeignetes Gefäß,
c) wobei die Schritte a) und b) so durchgeführt werden, daß das Gefäß 30 ml bis 70 ml Verdünnung mit einem Anteil von 1500 mg bis 3500 mg Esmolol oder des pharmazeutisch verträglichen Salzes, Solvates oder Derivates davon enthält,
d) Überführen des Gefäßes in eine verkaufsfertige Form.

2. Verfahren nach Anspruch 1, **dadurch gekennzeichnet, daß** das Gefäß 30 ml Verdünnung mit einem Anteil von 1500 mg Esmolol oder des pharmazeutisch verträglichen Salzes, Solvates oder Derivates davon enthält.

3. Verfahren nach Anspruch 1, **dadurch gekennzeichnet, daß** das Gefäß 50 ml Verdünnung mit einem Anteil von 2500 mg Esmolol oder des pharmazeutisch verträglichen Salzes, Solvates oder Derivates davon enthält.

4. Verfahren nach Anspruch 1, **dadurch gekennzeichnet, daß** das Gefäß 70 ml Verdünnung mit einem Anteil von 3500 mg Esmolol oder des pharmazeutisch verträglichen Salzes, Solvates oder Derivates davon enthält.
